# FASCICULE DE BREVET EUROPEEN

(11) **EP 0 988 391 B1**
(45) Date de publication et mention de la délivrance du brevet: **15.12.2004**
(21) Numéro de dépôt: 98928412.0
(22) Date de dépôt: 02.06.1998
(51) Int. Cl.: C12N 15/86, C12N 5/10, A61K 48/00, C12N 15/26, C07K 14/55, C12N 15/12, C07K 14/745

(54) **VECTEURS ADENOVIRAUX RECOMBINANTS COMPRENANT UNE SEQUENCE D'EPISSAGE**
REKOMBINANTE ADENOVIRALE VEKTOREN, DIE EINE SPLEISSEQUENZ ENTHALTEN
RECOMBINANT ADENOVIRAL VECTORS COMPRISING A SPLICING SEQUENCE

(30) Priorité: 02.06.1997 FR 9706757
(43) Date de publication de la demande: 29.03.2000
(73) Titulaire: TRANSGENE S.A., 67000 Strasbourg (FR)
(72) Inventeur: MEHTALI, Majid, F-67400 Illkirch Graffenstaden (FR); LEROY, Pierre, F-67000 Strasbourg (FR); MICHOU, Anne-Isabelle, CH 4058 Bale (CH)
(74) Mandataire: Warcoin, Jacques
(86) Numéro de dépôt international: PCT/FR1998/001105
(87) Numéro de publication internationale: WO 1998/055639

(56) Documents cités:
- EP-A- 0 732 405
- WO-A-94/12649
- WO-A-94/29471
- WO-A-95/16772
- WO-A-96/33280
- WO-A-97/04119
- US-A- 5 518 913
- Promega Catalogue 1996 page 213 Vecteur pCI XP002084598 cité dans la demande
- GREEN S. ET AL.: "A versatile in vivo and in vitro eukaryotic expression vector for protein engineering" NUCLEIC ACIDS RESEACH, vol. 16, no. 1, 1988, page 369 XP002084153 cité dans la demande
- CONNELLY S. ET AL.: "High-level tissue specific expression of functional human factor VIII in mice" HUMAN GENE THERAPY, vol. 7, 20 janvier 1996, pages 183-195, XP002055414 cité dans la demande
- KURACHI S. ET AL.: "Role of intron I in expression of the human factor IX gene" THE JOURNAL OF BIOLOGICAL CHEMISTRY, vol. 270, no. 10, 10 mars 1995, pages 5276-5281, XP002055415
- KARASUYAMA H. ET MELCHERS F.: "Establishment of mouse cell lines which constitutively secrete large quantities of interleukin 2, 3, 4 or 5, using modified cDNA expression vectors" EUROPEAN JOURNAL OF IMMUNOLOGY, vol. 18, no. 1, 1 janvier 1988, pages 97-104, XP000565567 cité dans la demande

### LISTE DE SEQUENCES

### (1) INFORMATION GENERALE:

(i) DEPOSANT:
   (A) NOM: Transgene S.A.
   (B) RUE: 11 rue de Molsheim
   (C) VILLE: Strasbourg
   (E) PAYS: France
   (F) CODE POSTAL: 67082
   (G) TELEPHONE: 03 88 27 91 00
   (H) TELECOPIE: 03 88 27 91 11
(ii) TITRE DE L' INVENTION: Nouveaux vecteurs adenoviraux recombinants comprenant une sequence d'epissage
(iii) NOMBRE DE SEQUENCES: 2
(iv) FORME LISIBLE PAR ORDINATEUR:
   (A) TYPE DE SUPPORT: Floppy disk
   (B) ORDINATEUR: IBM PC compatible
   (C) SYSTEME D' EXPLOITATION: PC-DOS/MS-DOS
   (D) LOGICIEL: PatentIn Release #1.0, Version #1.25 (OEB)

### (2) INFORMATION POUR LA SEQ ID NO: 1:

(i) CARACTERISTIQUES DE LA SEQUENCE:
   (A) LONGUEUR: 250 paires de bases
   (B) TYPE: acide nucléique
   (C) NOMBRE DE BRINS: simple
   (D) CONFIGURATION: linéaire
(ii) TYPE DE MOLECULE: ADN (génomique)
(iii) HYPOTHETIQUE: NON
(iii) ANTI-SENS: NON
(vi) ORIGINE:
   (C) INDIVIDUEL ISOLE: séquence d'epissage synthétique
(xi) DESCRIPTION DE LA SEQUENCE: SEQ ID NO: 1:
   CTGCAGAAGT TGGTCGTGAG GCACTGGGCA GGTAAGTATC AAGGTTACAA GACAGGTTTA      60
   AGGAGACCAA TAGAAACTGG GCTTGTCGAG ACAGAGAAGA CTCTTGCGTT TCTGATAGGC     120
   ACCTATTGGT CTTACTGACA TCCACTTTGC CTTTCTCTCC ACAGGTGTCC ACTCCCAGTT     180
   CAATTACAGC TCTTAAGGCT AGAGTACTTA ATACGACTCA CTATAGGCTA GCCTCGAGGT     240
   CGACCTGCAG                                                            250

### (2) INFORMATION POUR LA SEQ ID NO: 2:

(i) CARACTERISTIQUES DE LA SEQUENCE:
   (A) LONGUEUR: 652 paires de bases
   (B) TYPE: acide nucléique
   (C) NOMBRE DE BRINS: simple
   (D) CONFIGURATION: linéaire
(ii) TYPE DE MOLECULE: ADN (génomique)
(iii) HYPOTHETIQUE: NON
(iii) ANTI-SENS: NON
(vi) ORIGINE:
   (C) INDIVIDUEL ISOLE: intron 2 beta globine de lapin
(xi) DESCRIPTION DE LA SEQUENCE: SEQ ID NO: 2:
   GTCGACCGAT CCTGAGAACT TCAGGGTGAG TTTGGGGACC CTTGATTGTT CTTTCTTTTT     60
   CGCTATTGTA AAATTCATGT TATATGGAGG GGGCAAAGTT TTCAGGGTGT TGTTTAGAAT    120
   GGGAAGATGT CCCTTGTATC ACCATGGACC CTCATGATAA TTTTGTTTCT TTCACTTTCT    180
   ACTCTGTTGA CAACCATTGT CTCCTCTTAT TTTCTTTTCA TTTTCTGTAA CTTTTTCGTT    240
   AAACTTTAGC TTGCATTTGT AACGAATTTT TAAATTCACT TTTGTTTATT TGTCAGATTG    300
   TAAGTACTTT CTCTAATCAC TTTTTTTTCA AGGCAATCAG GGTATATTAT ATTGTACTTC    360
   AGCACAGTTT TAGAGAACAA TTGTTATAAT TAAATGATAA GGTAGAATAT TTCTGCATAT    420
   AAATTCTGGC TGGCGTGGAA ATATTCTTAT TGGTAGAAAC AACTACACCC TGGTCATCAT    480
   CCTGCCTTTC TCTTTATGGT TACAATGATA TACACTGTTT GAGATGAGGA TAAAATACTC    540
   TGAGTCCAAA CCGGGCCCCT CTGCTAACCA TGTTCATGCC TTCTTCTCTT TCCTACAGCT    600
   CCTGGGCAAC GTGCTGGTTG TTGTGCTGTC TCATCATTTT GGCAAAGAAT TC             652

## Description

La présente invention concerne des vecteurs adénoviraux comportant une cassette d'expression d'un gène d'intérêt placé sous le contrôle des éléments nécessaires à son expression et comprenant des séquences d'épissage. Leur présence permet d'augmenter sensiblement l'expression du gène thérapeutique dans une cellule ou un organisme hôte. Elle a également pour objet les cellules et les particules virales infectieuses contenant ces nouveaux vecteurs ainsi qu'une méthode pour les préparer. L'invention présente un intérêt tout particulier pour des perspectives de thérapie génique, notamment chez l'homme.

La thérapie génique se définit comme le transfert d'information génétique dans une cellule ou un organisme hôte. Le premier protocole appliqué à l'homme a été initié aux Etats-Unis en septembre 1990 sur un patient génétiquement immunodéficient en raison d'une mutation affectant le gène codant pour l'Adénine Désaminase (ADA). Le succès relatif de cette première expérimentation a encouragé le développement de cette technologie pour diverses maladies aussi bien génétiques (dans le but de corriger le dysfonctionnement d'un gène défectueux) qu'acquises (maladies infectieuses, cancers...). A l'heure actuelle, la majorité des protocoles mettent en oeuvre des vecteurs rétroviraux pour transférer et exprimer le gène thérapeutique dans les cellules à traiter. Cependant, outre leur capacité restreinte de clonage, ils présentent deux inconvénients majeurs qui limitent leur utilisation systématique : d'une part, ils infectent majoritairement les cellules en division et d'autre part, du fait de leur intégration au hasard dans le génome de la cellule hôte, le risque de mutagénèse insertionnelle n'est pas négligeable. C'est pourquoi, de nombreuses équipes scientifiques se sont attachées à développer d'autres types de vecteurs, parmi lesquels les adénovirus.

Mis en évidence dans de nombreuses espèces animales, les adénovirus sont peu pathogènes, non intégratifs et se repliquent aussi bien dans les cellules en division que quiescentes. De plus, ils présentent un large spectre d'hôte et sont capables d'infecter un grand nombre de types cellulaires, notamment les cellules épithéliales, endothéliales, les myocytes, les hépatocytes, les cellules nerveuses et les synoviocytes. En outre, ils possèdent un tropisme naturel pour les voies respiratoires. Ces propriétés particulières font des adénovirus des vecteurs de choix pour de nombreuses applications thérapeutiques et même vaccinales.

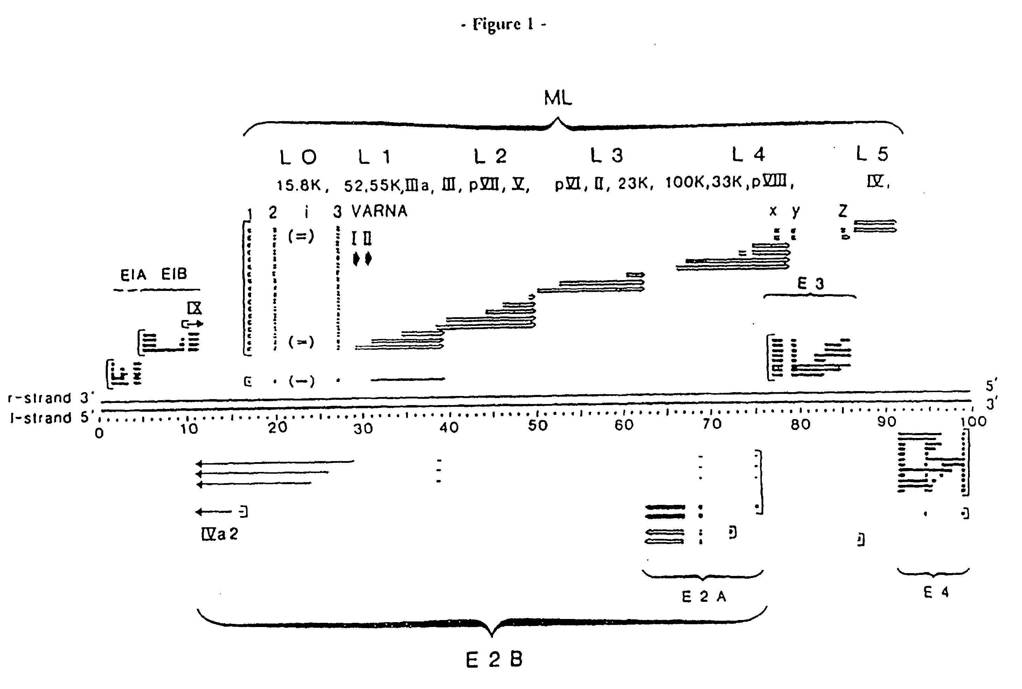

D'une manière générale, le génome adénoviral est constitué d'une molécule d'ADN linéaire et bicaténaire et d'environ 36 kb portant plus d'une trentaine de gènes codant pour les protéines virales et à ses extrémités deux répétitions inversées (désignées ITR pour Inverted Terminal Repeat) et la région d'encapsidation. Les gènes précoces sont répartis en 4 régions dispersées dans le génome (E1 à E4 ; E pour early en anglais) comportant 6 unités transcriptionnelles munies de leur propre promoteur. Les gènes tardifs (L1 à L5 ; L pour late signifiant tardif en anglais) codant pour les protéines de structure, recouvrent en partie les unités de transcription précoces et sont, pour la plupart, transcrits à partir du promoteur majeur tardif MLP (pour Major Late Promoter en anglais) (voir Figure 1).

Les vecteurs adénoviraux actuellement utilisés dans les protocoles de thérapie génique sont des vecteurs dits de première génération dépourvus de la majeure partie de la région E1 essentielle à la replication, afin d'éviter leur dissémination dans l'environnement et l'organisme hôte. La délétion de la région E3 non essentielle permet d'accroître leur capacité de clonage. Les gènes d'intérêt sont introduits dans l'ADN viral à la place de l'une ou l'autre région délétée. Ces virus défectifs pour la replication peuvent être propagés dans une lignée cellulaire complémentant la fonction E1. On utilise couramment la lignée 293, établie à partir de cellules de rein embryonnaire humain (Graham et al., 1977, J. Gen. Virol. *36*, 59-72). La délétion de la région E3 non essentielle ne nécessite pas de complémentation particulière. Si la faisabilité du transfert de gènes en utilisant ces vecteurs de première génération est maintenant bien établie, la question de leur innocuité reste posée. Outre les aspects de sécurité (risque de générer des particules compétentes pour la replication), se pose le problème de leur toxicité. En effet, les premiers essais cliniques ont mis en évidence l'induction de réponses inflammatoires dues à l'expression des gènes viraux chez l'hôte s'opposant à la persistance des cellules transduites et à l'expression du transgène. Ces inconvénients liés à la stimulation du système immunitaire de l'hôte par les épitopes adénoviraux ont justifié la construction de virus de nouvelles générations.

La conception d'un vecteur adénoviral repose d'une part sur le squelette viral et, d'autre part, sur la cassette d'expression du gène thérapeutique associé à des éléments de régulation permettant une expression optimale dans la cellule hôte. Sur le premier point, les vecteurs adénoviraux de seconde génération conservent les régions *en cis* ITRs et séquences d'encapsidation et comportent des délétions internes importantes visant à supprimer l'essentiel des gènes viraux dont l'expression *in vivo* n'est pas souhaitable (voir la demande internationale WO94/28152). Leur propagation est assurée par l'intermédiaire d'un virus auxiliaire ou de lignées cellulaires complémentant les fonctions défectives. Par exemple, on utilisera une lignée dérivée de la 293 et exprimant les séquences adénovirales codant pour les protéines essentielles de E4 pour complémenter un vecteur de seconde génération dont le squelette génomique est délété des régions E1, E3 et E4.

Pour ce qui est de la cassette d'expression, celle-ci comprend généralement en 5' une région promotrice dirigeant la transcription du gène situé à sa suite et, éventuellement en 3', une séquence de polyadénylation (polyA) qui contribue notamment à stabiliser le messager transcrit. Des éléments additionnels peuvent dans certains contextes améliorer l'expression. L'effet positif des séquences introniques sur l'expression génique à déjà été reporté *in vitro* (Buchman et Berg, 1988, Mol. Cell. Biol. *8*,4395-4405; Huang et Gorman, 1990, Nucleic Acid Res. *18*, 937-947), *in vivo* dans les animaux transgéniques (Brinster et al., 1988, Proc Natl. Acad. Sci. USA *85,* 836-840) et plus récemment dans un contexte vecteur adénoviral de première génération (Connelly et al., 1996, Human Gene Therapy 7, 183-195). Ce document démontre que les souris traitées par un adénovirus délété des régions E1 et E3 et exprimant le facteur VIII humain produisent des niveaux 3 à 13 fois plus élevés de facteur VIII sérique lorsque l'ADN complémentaire FVIII comporte des séquences d'épissage.

On peut également citer la demande internationale de brevet publiée sous le numéro WO 94/29471 le 22 Décembre 1994 qui décrit un vecteur adénoviral recombinant contenant un gène d'intérêt codant pour le facteur de coagulation FIX sous le contrôle d'un promoteur et d'une séquence de polyadénylation. L'addition d'une séquence d'épissage homologue issue du gène FIX permet d'accroître substantiellement le niveau de production du FIX, essentiellement en présence des séquences 5' et 3' non codantes de ce gène.

La présente invention a pour but de mettre à la disposition du public des vecteurs adénoviraux plus efficaces du point de vue de l'expression du gène thérapeutique permettant ainsi de réduire les doses de vecteurs et d'amplifier l'effet thérapeutique. On a maintenant montré que la présence de séquences d'épissage au sein du gène d'intérêt est bénéfique voire indispensable pour obtenir son expression. Cette observation est particulièrement vraie dans un contexte vecteur adénoviral de seconde génération où les niveaux d'expression des gènes thérapeutiques facteur IX (FIX) canin et interleukine-2 (IL-2) humaine, sont amplifiés d'un facteur 20 à 150 lorsque la cassette d'expression inclut lesdites séquences d'épissage. Avec un vecteur de première génération, le facteur d'amplification reste significatif (2 à 3). Cette amélioration importante de l'expression génique est inattendue et ne pouvait être déduite de l'état de la technique.

C'est pourquoi la présente invention a pour objet un vecteur adénoviral dérivant du génome d'un adénovirus par délétion d'au moins tout ou partie de la région E1, ledit vecteur adénoviral comportant une cassette d'expression d'un gène d'intérêt placé sous le contrôle des éléments nécessaires à son expression dans une cellule hôte ou un organisme hôte, lesdits éléments nécessaires à l'expression comprennant au moins une séquence d'épissage, caractérisé en ce que ladite séquence d'épissage comprend un site donneur et un site accepteur d'épissage et dérive d'un gène β-globine, ou d'une séquence d'épissage synthétique ayant la séquence représentée à l'identificateur de séquence IDS1.

Au sens de la présente invention, le terme vecteur adénoviral désigne un adénovirus défectif pour la replication (incapable de replication autonome dans une cellule hôte) en l'absence de toute complémentation. Le vecteur selon l'invention est modifié par rapport à l'adénovirus parental au moins dans la région E1 par délétion totale ou partielle de cette dernière. Selon un mode de réalisation avantageux, en outre l'une au moins des régions sélectionnées parmi E2, E4, L1, L2, L3, L4 et L5 est non fonctionnelle. La non fonctionnalité peut être obtenue par délétion totale ou partielle d'une ou plusieurs des régions concernées ou par introduction de mutation(s) (délétion, addition et/ou substitution d'un ou plusieurs nucléotides) rendant le gène adénoviral muté défectif. Ces modifications peuvent toucher les séquences codantes du génome viral ou non codantes, notamment les régions promotrices. Pour illustrer ces modes de réalisation, on peut citer la mutation thermosensible affectant le gène DBP (pour DNA Binding Protein en anglais) de la région E2A (Ensinger et al., 1972, J. Virol. *10*, 328-339). Une délétion partielle peut consister en l'élimination de la région E4 à l'exception des séquences codant pour les cadres de lecture ouverts (ORF) 6 et 7, qui ne nécessitent pas de complémentation de la fonction E4 (Ketner et al., 1989, Nucleic Acids Res. *17*, 3037-3048). Une délétion totale de E4 couvre l'unité transcriptionnelle complète.

On indique qu'un vecteur adénoviral selon l'invention conserve les régions *en cis* du génome adénoviral à savoir les répétitions inversées terminales (ITR) et la région d'encapsidation. Leur longueur et séquence nucléotidique peuvent varier d'un sérotype à l'autre. Cependant, elles peuvent être aisément isolées à partir des données de la littérature. A titre indicatif, les 458 premiers nucléotides (nt) du génome de l'adénovirus de type 5 (Ad5) portent l'IRT 5' et la région d'encapsidation et les 103 derniers nt correspondent à l'ITR 3'. Avantageusement, le vecteur adénoviral selon l'invention comprend également les séquences codant pour la protéine pIX à moins qu'elles ne soient complémentées par la lignée de production. Par ailleurs, il peut en outre être dépourvu de tout ou partie de la région E3. Une autre alternative consiste à conserver les séquences E3 codant pour les polypeptides permettant l'échappement au système immunitaire de l'hôte, notamment la glycoprotéine gp19k (Gooding et al., 1990, Critical Review of Immunology *10*, 53-71). Dans le cadre de la présente invention, on peut avoir recours aux vecteurs dits de seconde génération de l'état de la technique (voir par exemple les demandes internationales WO94/28152 et WO97/04119).

Un vecteur adénoviral selon l'invention peut également être modifié aux niveau des séquences codant pour les protéines tardives, comme l'hexon, le penton ou la fibre afin de modifier l'infectivité du virion par exemple pour cibler un type cellulaire particulier (voir par exemple la demande française FR97 04747).

Un vecteur adénoviral préféré selon l'invention est choisi parmi les suivants :
(1) vecteur adénoviral dépourvu de tout ou partie des régions E1 et E2 et, de manière optionnelle, de tout ou partie de E3,
(2) vecteur adénoviral dépourvu de tout ou partie des régions E1 et E4 et, de manière optionnelle, de tout ou partie de E3,
(3) vecteur adénoviral dépourvu de tout ou partie des régions E1 et E4 et, de manière optionnelle, de tout ou partie de E3, et comportant une mutation non fonctionnelle dans la région E2,
(4) vecteur adénoviral dépourvu de tout ou partie des régions E1, E2 et E4 et, de manière optionnelle de tout ou partie de E3,
(5) vecteur adénoviral dépourvu de tout ou partie de la région E1 et, de manière optionnelle de tout ou partie de E3.

L'origine du vecteur adénoviral selon l'invention, peut être variée aussi bien du point de vue de l'espèce que du sérotype. Il peut dériver du génome d'un adénovirus d'origine humaine, canine, aviaire, bovine, murine, ovine, porcine ou simienne ou encore d'un hybride comprenant des fragments de génome adénoviral de différentes origines. On peut citer plus particulièrement les adénovirus CAV-1 ou CAV-2 d'origine canine, DAV d'origine aviaire ou encore Bad de type 3 d'origine bovine (Zakharchuk et al., Arch. Virol., 1993, *128*: 171 - 176 Spibey et Cavanagh, J. Gen. Virol., 1989, *70*: 165-172 ; Jouvenne et al., Gene, 1987, *60*: 21-28 ; Mittal et al., J. Gen. Virol., 1995, *76*: 93-102). Cependant, on préférera un vecteur adénoviral d'origine humaine dérivant de préférence d'un adénovirus de sérotype C, notamment de type 2 ou 5. Par ailleurs, le vecteur adénoviral selon la présente invention peut être généré *in vitro* dans *Escherichia coli (E. coli)* par les techniques de biologie moléculaire ou encore par recombinaison homologue (voir par exemple la demande internationale WO96/17070).

Comme indiqué précédemment, le vecteur adénoviral selon l'invention est recombinant et comporte au moins une cassette d'expression d'un gène d'intérêt placé sous le contrôle des éléments nécessaires à son expression dans une cellule hôte ou un organisme hôte. Préférentiellement, elle est insérée dans le vecteur adénoviral selon l'invention en remplacement d'une des régions délétées, notamment de E1. Dans le cas où l'on met en oeuvre plusieurs cassettes d'expression, elles peuvent être insérées au même endroit ou à des endroits différents du génome viral, utiliser les mêmes éléments de régulation ou des éléments différents et, éventuellement, être en orientation réverse les unes par rapport aux autres afin de minimiser les phénomènes d'interférence au niveau de l'expression génique.

La caractéristique essentielle de l'une au moins des cassettes d'expression mises en oeuvre dans le cadre de la présente invention, est de comprendre au moins une séquence d'épissage. Le terme "séquence d'épissage" désigne une séquence habituellement active dans l'épissage d'une séquence intervenante (ivs) située entre deux points d'épissage, présente dans un gène nucléaire entre deux exons et absente de l'ARN messager (ARNm) correspondant. Les exons sont les segments de séquence qui constituent l'ARNm. Ils peuvent être codants ou non-codants, notamment ceux localisés aux extrémités 5' et 3'. Les points d'épissage représentent les points frontières entre exon et ivs, le point donneur étant au début de l'ivs et le point accepteur à la fin. Ladite séquence d'épissage comprend au moins les séquences situées directement en 3' du point donneur et en 5' du point accepteur d'épissage et éventuellement une séquence de taille quelconque les séparant. Elle peut également comporter à l'une ou l'autre ou ses deux extrémités des séquences supplémentaires. On mettra de préférence en oeuvre des séquences exoniques, non codantes, intervenant directement dans le processus d'épissage et comprenant les séquences directement en 5' du point donneur et en 3' du point donneur d'épissage. Ce mode de réalisation est particulièrement avantageux avec un gène d'intérêt de type ADN complémentaire (ADNc). Les séquences intervenant directement dans l'épissage (sites d'épissage recouvrant la jonction exon-ivs) sont relativement bien conservées dans l'évolution et les consensus divulgués dans la plupart des ouvrages de base traitant de l'expression des gènes eucaryotes (par exemple dans Watson et al., 1989, in Molecular Biology of the Gene, 4 ed, p 683-742 , Benjamin/Cummings Publishing Company Inc., Menlo Park, Californie). Notamment, les séquences GT et AG situées respectivement en aval et en amont des points d'épissage 5' (donneur) et 3' (accepteur) sont quasi invariables.

Il est connu que de nombreux gènes eucaryotes sont constitués par une succession d'exons et d'introns. Les séquences d'épissage susceptibles d'être mis en oeuvre dans la présente invention peuvent avoir des longueurs et des séquences bien différentes. Ils peut s'agir d'une séquence d'épissage native telle que trouvée dans la nature. On peut aussi employer une séquence d'épissage modifiée, notamment par la suppression d'une ou plusieurs séquences non actives dans le processus d'épissage, dans le but de réduire sa taille ou d'éliminer des séquences répétées pouvant conduire à des phénomènes de recombinaison ou des séquences de régulation susceptibles de perturber l'expression du gène d'intérêt. On peut envisager d'utiliser une séquence d'épissage chimère formée de séquences d'origines diverses. Pour illustrer cet aspect, l'intron chimère peut être formé des parties 5' et 3' de deux introns différents ou être muni d'un site donneur d'épissage et/ou d'un site accepteur d'épissage hétérologue. Il est également possible d'avoir recours à une séquence d'épissage synthétique conçu à partir des sites d'épissage consensus. Une séquence d'épissage préférée selon l'invention dérive du second intron du gène β-globine de lapin (Green et al., 1988, Nucleic Acid Res. *16*, 369 ; Karasuyama et al., 1988, Eur. J. Immunol. *18*, 97-104 ; Karasuyama et al., 1989, J. Exp. Med. *169*, 13-25), ou de celle trouvée dans le plasmide pCI (Promega Corp, pCI mammalian expression vector E1731) comprenant le site donneur d'épissage de l'intron 1 du gène β-globine humaine ainsi que le point de branchement et le site accepteur d'épissage du gène d'une immunoglobine de souris.

De manière préférée, un vecteur adénoviral selon l'invention comprend une séquence d'épissage identique à au moins 70% ou identique à la séquence représentée à l'identificateur de séquence IDS 1 ou 2. L'identité de séquence entre la séquence en usage dans la présente invention et celle reportée dans l'un ou l'autre des IDS est d'au moins 70 %, avantageusement d'au moins 80 %, de préférence d'au moins 90 % et, de manière tout à fait préférée d'au moins 95%. L'identité de séquence signifie 100 % d'identité et « sensiblement telle » désigne une identité de séquence d'au moins 95 %. Une partie comprend au moins 17 nt continus. Un vecteur adénoviral selon l'invention comprenant une séquence d'épissage sensiblement telle que représentée à l'identificateur de séquence IDS 1 ou 2 convient tout particulièrement.

Conforméments aux buts poursuivis par la présente invention, la cassette d'expression peut comporter une ou plusieurs séquences d'épissage insérées au sein d'un ou plusieurs gènes d'intérêt de type génomique, minigène (type mixte entre génomique et ADNc) ou encore ADNc (dépourvu d'intron) portés par ladite cassette. Le site d'insertion préférentiel de la séquence d'épissage au sein du gène d'intérêt est entre le premier exon et le second exon. Lorsque le gène d'intérêt est de type ADNc, on aura de préférence recours à une séquence d'épissage munie de courtes séquences exoniques pouvant être insérée en 5' ou en 3' de l'ADNc. Le gène d'intérêt peut être homologue ou hétérologue à la cellule hôte et coder pour un ARN antisens, un ribozyme ou un polypeptide d'intérêt de localisation nucléaire, cytoplasmique, membranaire ou secrété. Il peut s'agir d'un polypeptide natif tel que trouvé dans la nature, d'un fragment fonctionnel, d'un mutant présentant des propriétés biologiques améliorées et/ou modifiées ou encore d'une chimère provenant de la fusion de séquences d'origines diverses. Le gène d'intérêt peut être obtenu par synthèse chimique ou par clonage (criblage de banque d'ADN à l'aide de sondes appropriées, PCR...) et peut être modifié par les techniques conventionnelles de biologie moléculaire.

Dans le cadre de la présente invention, il peut être avantageux d'utiliser un gène d'intérêt codant pour une cytokine (interféron α, β ou γ, interleukine (IL), notamment l'IL-2, l'IL-6, l'IL-10 ou encore l'IL-12, un facteur nécrosant des tumeurs (TNF), un facteur stimulateur de colonies (GM-CSF, C-CSF, M-CSF...), un récepteur cellulaire (notamment reconnu par le virus HIV), un ligand de récepteur, un facteur de coagulation, un facteur de croissance (FGF pour Fibroblast Growth Factor, VEGF pour Vasculau Endothélial Growth Factor...), une enzyme (uréase, rénine, thrombine, métalloprotéinase, NOS pour Nitric Oxide synthétase, SOD, catalase....), un inhibiteur d'enzyme (α1-antitrypsine, antithrombine III, inhibiteur de protéase virale, PAI-1 pour plasminogen activator inhibitor...), un antigène du complexe majeur d'histocompatibilité de classe 1 ou II ou un polypeptide agissant sur l'expression des gènes correspondants, un polypeptide capable d'inhiber une infection virale, bactérienne ou parasitaire ou son développement, un polypeptide agissant positivement ou négativement sur l'apoptose (Bax, Bcl2, BclX...), un agent cytostatique (p21, p16, Rb...), une apolipoprotéine (ApoAI, ApoAIV, ApoE...), un inhibiteur d'angiogénèse (angiostatine, endostatine...), un marqueur (β-galactosidase, luciférase....) ou tout autre gène d'intérêt ayant un effet thérapeutique pour l'affection ciblée.

Plus précisemment, dans le but de traiter un dysfonctionnement héréditaire, on utilisera une copie fonctionnelle du gène défectueux, par exemple un gène codant pour le facteur VIII ou IX dans le cadre de l'hémophilie A ou B, la dystrophine dans le cadre des myopathies de Duchenne et Becker, l'insuline dans le cadre du diabète, la protéine CFTR (Cystic Fibrosis Transmembrane Conductance Regulator) dans le cadre de la mucoviscidose. S'agissant d'inhiber l'initiation ou la progression de tumeurs ou cancers, on mettra de préférence en oeuvre un gène d'intérêt codant pour un ARN anti-sens, un ribozyme, un produit cytotoxique (thymidine kinase de virus simplex de l'herpès 1 (TK-HSV-1), ricine, toxine cholérique, diphtérique, produit d'expression des gènes de levure *FCY1* et *FUR1* codant pour l'uracile phosphoribosyl transférase et la cytosine désaminase.....), un anticorps, un inhibiteur de la division cellulaire ou des signaux de transduction, un produit d'expression d'un gène suppresseur de tumeur (p53, Rb, p73....), un polypeptide stimulateur du système immunitaire, un antigène associé à une tumeur (MUC-1, BRCA-1, antigènes précoces ou tardifs (E6, E7, L1, L2...) d'un virus à papillome HPV....), éventuellement en combinaison avec un gène de cytokine. Enfin, dans le cadre d'une thérapie anti-HIV, on peut avoir recours à un gène codant pour un polypeptide immunoprotecteur, un épitope antigénique, un anticorps (2F5; Buchacher et al., 1992, Vaccines 92, 191-195), le domaine extracellulaire du récepteur CD4 (sCD4 ; Traunecker et al., 1988, Nature *331*, 84-86) une immunoadhésine (par exemple un hybride CD4-immunoglobuline IgG ; Capon et al., 1989, Nature *337*, 525-531 ; Byrn et al., 1990, Nature *344*, 667-670), une immunotoxine (par exemple fusion de l'anticorps 2F5 ou de l'immunoadhésine CD4-2F5 à l'angiogénine; Kurachi et al., 1985, Biochemistry *24*, 5494-5499), un variant trans-dominant, un produit cytotoxique tel que l'un de ceux mentionné ci-dessus ou encore un IFNα ou β.

Par ailleurs, la cassette d'expression en usage dans la présente invention peut également comprendre un gène de sélection permettant de sélectionner ou identifier les cellules transfectées. On peut citer les gènes néo (codant pour la néomycine phosphotransférase) conférant une résistance à l'antibiotique G418, *dhfr* (Dihydrofolate Réductase), CAT (Chloramphenicol Acetyl transférase), *pac* (Puromycine Acétyl-Transferase) ou encore *gpt* (Xanthine Guanine Phosphoribosyl Transferase). D'une manière générale, les gènes de sélection sont connus de l'homme de l'art.

La locution "éléments nécessaires à l'expression" désigne les éléments génétiques permettant la transcription d'un gène d'intérêt en ARN et la traduction d'un ARNm en polypeptide. Parmi ceux-ci, le promoteur revêt une importance particulière. Il peut être isolé d'un gène quelconque d'origine eucaryote ou même virale et peut être constitutif ou régulable. Alternativement, il peut s'agir du promoteur naturel du gène en question. Par ailleurs, il peut être modifié de manière à améliorer l'activité promotrice, supprimer une région inhibitrice de la transcription, rendre un promoteur constitutif régulable ou vice versa, introduire un site de restriction.... On peut mentionner, à titre d'exemples, les promoteurs viraux CMV (Cytomégalovirus), RSV (Rous Sarcoma Virus), du gène TK du virus HSV-1, précoce du virus SV40 (Simian Virus 40), adénoviral d'un gène précoce ou tardif (E1A, MLP...) ou encore les promoteurs eucaryotes des gènes PGK (Phospho Glycerate kinase), MT (métallothionéine), α1-antitrypsine, CFTR, surfactant (poumon-spécifique), immunoglobuline (lymphocyte-spécifique), actine (muscle-spécifique) ou encore SRα (hybride entre l'origine de SV40 et le LTR de HTLV-1 ; Takebe et al., 1988, Mol. Cell. Biol. *8*, 466-472). Il peut également s'agir d'un promoteur stimulant l'expression dans une cellule tumorale ou cancéreuse. On peut citer notamment les promoteurs des gènes MUC-1 surexprimé dans les cancers du sein et de la prostate (Chen et al., 1995, J. Clin. Invest. *96*, 2775-2782), CEA (pour carcinoma embryonic antigen) surexprimé dans les cancers du colon (Schrewe et al., 1990, Mol. Cell. Biol. *10*, 2738-2748), tyrosinose surexprimé dans les mélanomes (Vile et al., 1993, Cancer Res. *53*, 3860-3864), ERB-2 surexprimé dans les cancers du sein et du pancréas (Harris et al., 1994, Gene Therapy *1*, 170-175) et α-fétoprotéine surexprimée dans les cancers du foie (Kanai et al., 1997, Cancer Res. *57*, 461-465). Le promoteur précoce du Cytomégalovirus (CMV) est tout particulièrement préféré.

Lorsque la cassette d'expression en usage dans la présente invention comprend plusieurs gènes d'intérêt, ceux-ci peuvent être placés sous le contrôle des mêmes éléments génétiques (cassette polycistronique utilisant un site interne d'initiation de la traduction de type IRES pour réinitier la traduction du second cistron) ou d'éléments indépendants.

Bien entendu, la cassette peut en outre comprendre des éléments additionnels améliorant l'expression du gène d'intérêt (séquence signal, séquence de localisation nucléaire, séquence de polyadénylation, IRES, leader tripartite....) ou encore le maintien dans la cellule hôte (origine de réplication ...). De tels éléments sont connus de l'homme de l'art. Une séquence de polyadénylation préférée dérive du virus SV40 ou encore du gène β-globine de lapin.

Un mode de réalisation particulièrement avantageux réside en un vecteur adénoviral dont le génome est délété des régions E1, E3 et E4 dans lequel est insérée à la place de la région E1, une cassette d'expression comportant le promoteur CMV, la séquence d'épissage synthétique isolée du plasmide pCI, l'ADNc codant pour l'IL-2 humaine et le polyA du virus SV40 (tel que pTG6215). Une autre variante intéressante est fournie par un vecteur adénoviral de squelette génomique similaire (délétion E1, E3 et E4) comprenant une cassette d'expression formée du promoteur RSV suivi des séquences d'épissage comprenant l'intron 2 du gène β-globine de lapin, de l'ADNc du facteur IX canin et du poly A du gène β-globine de lapin (tel que pTG9378). Un autre exemple préféré consiste en un vecteur E1⁻ E3⁻ dans lequel est insérée une cassette le promoteur CMV, l'intron synthétique de pCI, l'ADNc codant pour l'IL-2 humaine suivi du polyA de SV40 (tel que pTG6624).

L'invention a également trait à une particule virale infectieuse ainsi qu'à une cellule hôte eucaryote comprenant un vecteur adénoviral selon l'invention. Ladite cellule hôte est avantageusement une cellule de mammifère et, de préférence, une cellule humaine et peut comprendre ledit vecteur sous forme intégrée ou non dans le génome. Il peut s'agir d'une cellule primaire ou tumorale d'une origine hématopoïétique (cellule souche totipotente, leucocyte, lymphocyte, monocyte ou macrophage ...), musculaire (cellule satellite, myocyte, myoblaste ...), cardiaque, hépatique, pulmonaire, trachéale, épithéliale ou fibroblaste. Une particule virale infectieuse selon l'invention est préparée selon toute technique conventionnelle dans le domaine de l'art (Graham et Prevect, 1991, *supra*). Plus précisemment, le vecteur adénoviral selon l'invention est propagé dans une lignée de complémentation capable de fournir *en trans* les fonctions défectueuses afin de produire les polypeptides nécessaires à la constitution des particules virales infectieuses. On aura notamment recours aux lignées décrites dans les demandes internationales WO94/28152 et WO 97/04119. On peut également employer une lignée cellulaire appropriée, comme la lignée 293 pour complémenter la fonction E1 (Graham et al., 1977, *supra*) ou A549-E1 (WO94/28152). Pour une complémentation multiple, il est également possible de mettre en oeuvre un virus auxilliaire ou une lignée de complémentation de l'état de la technique (par exemple Lusky et al., 1998, J. Virol 72, 2022-2033).

L'invention concerne également un procédé de préparation d'une particule virale infectieuse comprenant un vecteur adénoviral selon l'invention, selon lequel :
(i) on introduit ledit vecteur adénoviral selon l'invention dans une cellule de complémentation capable de complémenter *en trans* ledit vecteur, de manière à obtenir une cellule de complémentation transfectée,
(ii) on cultive ladite cellule de complémentation transfectée dans des conditions appropriées pour permettre la production de ladite particule virale infectieuse, et
(iii) on récupère ladite particule virale infectieuse dans la culture cellulaire.

Bien entendu, la particule virale infectieuse peut être récupérée du surnageant de culture mais également des cellules. Une des méthodes couramment employée consiste à lyser les cellules par des cycles consécutifs de congélation/décongélation pour recueillir les virions dans le surnageant de lyse. Ceux-ci peuvent être amplifiés et purifiés selon les techniques de l'art (procédé chromatographique, ultracentrifugation notamment à travers un gradient de chlorure de césium...).

L'invention a également pour objet une composition pharmaceutique comprenant à titre d'agent thérapeutique ou prophylactique, un vecteur adénoviral, une particule virale infectieuse ou une cellule hôte eucaryote selon l'invention ou encore une particule virale infectieuse susceptible d'être obtenue par un procédé de préparation de particule virale infectieuse selon l'invention, en association avec un support acceptable d'un point de vue pharmaceutique. La composition selon l'invention est, en particulier, destinée au traitement préventif ou curatif de maladies génétiques (hémophilie, diabète, mucoviscidose, myopathie de Duchenne ou de Becker, maladies autoimmunes...), de cancers et tumeurs, de maladies virales (l'hépatite B ou C, SIDA, infections herpétiques .... ), de maladies cardiovasculaires (resténoses...etc).

Une composition pharmaceutique selon l'invention peut être fabriquée de manière conventionnelle en vue d'une administration par voie locale, parentérale ou digestive. En particulier, on associe une quantité thérapeutiquement efficace de l'agent thérapeutique ou prophylactique à un support acceptable d'un point de vue pharmaceutique. Les voies d'administration envisageables sont multiples. On peut citer par exemple la voie intragastrique, sous-cutanée, intracardiaque, intramusculaire, intraveineuse, intrapéritonéale, intratumorale, intranasale, intrapulmonaire ou intratrachéale. Pour ces trois derniers modes de réalisation, une administration par aérosol ou instillation est avantageuse. L'administration peut avoir lieu en dose unique ou répétée, une ou plusieurs fois après un certain délai d'intervalle. La voie d'administration et le dosage appropriés varient en fonction de divers paramètres, par exemple, de l'individu ou de la maladie à traiter ou encore du ou des gène(s) d'intérêt à transférer. En particulier, les particules virales selon l'invention peuvent être formulées sous forme de doses comprises entre 10⁴ et 10¹⁴ ufp (unités formant des plages), avantageusement 10⁵ et 10¹³ ufp et, de préférence, 10⁶ et 10¹² ufp. La formulation peut également inclure un diluant, un adjuvant ou un excipient acceptable d'un point de vue pharmaceutique. Elle peut être présentée sous forme liquide ou sèche (lyophilisat...).

Le vecteur et les particules virales virale selon l'invention peuvent être éventuellement associée à une ou plusieurs substances améliorant l'efficacité transfectionnelle et/ou la stabilité. Ces substances sont largement documentées dans la littérature accessible à l'homme de l'art (voir par exemple Felgner et al., 1987, Proc. West. Pharmacol. Soc. *32*, 115-121 ; Hodgson et Solaiman, 1996, Nature Biotechnology *14*, 339-342; Remy et al., 1994, Bioconjugate Chemistry *5*, 647-654). A titre illustratif mais non limitatif, il peut s'agir de polymères, de lipides notamment cationiques, de liposomes, de protéines nucléaires ou encore de lipides neutres. Ces substances peuvent être utilisées seules ou en combinaison.

Enfin, la présente invention est relative à l'utilisation d'un vecteur adénoviral, d'une particule virale infectieuse ou d'une cellule hôte eucaryote selon l'invention ou encore une particule virale infectieuse susceptible d'être obtenue par un procédé de préparation de particule virale infectieuse selon l'invention, pour le transfert d'un gène d'intérêt dans une cellule ou un organisme hôte. Selon une première possibilité, le médicament peut être administré directement *in vivo* (par exemple par injection intraveineuse, intramusculaire, dans une tumeur accessible, dans les poumons par aérosol...). On peut également adopter l'approche *ex vivo* qui consiste à prélever des cellules du patient (cellules souches de la moëlle osseuse, lymphocytes du sang périphérique ...), de les transfecter ou infecter *in vitro* selon les techniques de l'art et de les réadminister au patient. Il est décrit également la préparation d'un médicament destiné au traitement du corps humain ou animal par thérapie génique.

Enfin la présente invention a également pour objet l'utilisation d'un vecteur adénoviral selon l'invention, pour améliorer l'expression d'un gène d'intérêt d'un facteur au moins 20, avantageusement d'au moins 50 et, de préférence d'au moins 100 dans une cellule ou un organisme hôte. Le niveau d'amélioration peut être facilement déterminé en comparant l'expression du gène d'intérêt en présence et en absence de ladite séquence d'épissage dans un contexte adénoviral donné.

Il est décrit également une méthode de traitement selon laquelle on administre une quantité thérapeutiquement efficace d'un vecteur adénoviral, d'une particule virale ou d'une cellule hôte eucaryote selon l'invention à un patient ayant besoin d'un tel traitement.

La Figure 1 est une représentation schématique du génome de l'adénovirus humain de type 5 (représenté en unités arbitraires de 0 à 100) indiquant l'emplacement des différents gènes.

### EXEMPLES

La présente invention est illustrée, sans pour autant être limitée, par les exemples suivants.

Les constructions décrites ci-dessous sont réalisées selon les techniques générales de génie génétique et de clonage moléculaire, détaillées dans Maniatis et al., (1989, Laboratory Manual, Cold Spring Harbor, Laboratory Press, Cold Spring Harbor, NY) ou selon les recommandations du fabricant lorsqu'on utilise un kit commercial. Les étapes de recombinaison homologue sont de préférence réalisées dans la souche *E*. *coli* BJ 5183 (Hanahan, 1983, J. Mol. Biol. *166*, 557-580). S'agissant de la réparation des sites de restriction, la technique employée consiste en un remplissage des extrémités 5' protubérantes à l'aide du grand fragment de l'ADN polymérase I d'*E. coli* (Klenow). Les techniques d'amplification par PCR (Polymérase Chain Reaction) sont connues de l'homme de l'art (voir par exemple PCR Protocols - A Guide to Methods and Applications, 1990, édité par Innis, Gelfand, Sninsky et White, Academic Press Inc). Par ailleurs, les fragments de génome adénoviral employés dans les différentes constructions décrites ci-après, sont indiqués précisément selon leur position dans la séquence nucléotidique du génome de l'Ad5 telle que divulguée dans la banque de données Genebank sous la référence M73260.

En ce qui concerne la biologie cellulaire, les cellules sont transfectées ou transduites et cultivées selon les techniques standards bien connues de l'homme du métier. Dans les exemples qui suivent, on a recours aux lignées cellulaires 293 (Graham et al, 1977, *supra ;* disponible à l'ATCC sous la référence CRL1573), LCA-1 (correspondant au clone 5606#5-38 décrit à l'exemple 3 de la demande WO94/04119), A549 d'origine épithéliale et dérivant d'un carcinome pulmonaire (ATCC CCL-185) et A549-E1 (exemple 6 de WO94/28156). Il est entendu que d'autres lignées cellulaires peuvent être utilisées. On indique que la lignée A549-E1 est une lignée de complémentation de la fonction adénovirale E1 obtenue par transfection d'un plasmide portant les séquences E1 d'Ad5 (nt 505 à 4034) exprimées à partir du promoteur PGK. Les clones stables sélectionnés à la puromycine sont testés pour leur capacité de complémentation et on sélectionne le meilleur clone producteur (#73).

### EXEMPLE 1 : Construction du vecteur adénoviral AdTG6215 exprimant le gène de l'interleukine 2 humaine.

On isole du plasmide pCI (Promega) le fragment *Bgl*II*-Bam*HI portant le promoteur CMV, la séquence d'épissage, un site multiple de clonage (MCS) et la séquence polyA du virus SV40, lequel est inseré dans un plasmide conventionnel. Les séquences ADNc codant pour l'IL-2 humaine (Taniguchi et al., 1983, Nature *302,* 305-311) sont isolées sous forme d'un fragment *Xho*I*-EcoR*I (éventuellement par PCR) et introduites au niveau du MCS. La cassette est clonée en lieu et place de la région adénovirale E1 dans un vecteur de transfert adénoviral, pour donner pTG6601. Le vecteur de transfert comporte les nt 1 à 458 et 3329 à 6241 de l'Ad5 dans un plasmide ppolyII. On génère pTG6215 par recombinaison homologue entre le fragment *Pac*I*-BstE*II isolé de pTG6601 et le vecteur pTG8595 (Chartier et al., 1996, J. Virol. 70, 4805-4810) linéarisé par *Cla*I*.* Le vecteur adénoviral pTG6215 contient le génome Ad5 dépourvu des régions E1 (nt 459 à 3327), E3 (nt 28592 à 30470) et E4 (nt 32994 à 34998) et la cassette "promoteur CMV-séquence d'épissage de pCI-ADNc IL-2 et pA SV40" insérée à la place des séquences adénovirales E1.

A titre de témoin, on génère le vecteur pTG6692 en délétant les séquences d'épissage du bloc d'expression isolé de pCI par digestion *Pst*I et religation. Le clonage de l'ADNc IL-2 et l'insertion de la cassette "sans intron" au sein du génome adénoviral sont réalisés comme indiqué ci-dessus.

Enfin, il est utile de comparer l'effet des séquences d'épissage dans un contexte vecteur adénoviral de première génération. Pour ce faire, le vecteur pTG6624 est construit par recombinaison homologue entre le fragment *Pac*I*-Bst*EII isolé de pTG6601 et le vecteur pTG4656 linéarisé par *Cla*I*.* Ce dernier est équivalent à pTG8595 à la différence qu'il porte une région E4 intègre, de sorte que la construction finale pTG6624 correspond au génome Ad5 délété des régions E1 (nt 459 à 3327) et E3 (nt 28592 à 30470) avec la cassette "promoteur CMV-séquence d'épissage de pCI-ADNc IL-2 et pA SV40" insérée à la place de E1. Le vecteur "sans intron" de première génération désigné pTG6229, résulte de la délétion des séquences d'épissage par digestion *Pst*I et du clonage de la cassette sans intron dans le génome adénoviral par recombinaison homologue avec le vecteur pTG4656.

Les adénovirus AdTG6215 et AdTG6692 sont obtenus par transfection du fragment *Pac*I isolé des vecteurs correspondants dans les cellules LCA1 par la technique au phosphate de calcium. Les virions de première génération AdTG6624 et AdTG6229 sont produits dans la lignée 293. Les virus sont isolés, propagés et purifiés dans les conditions habituelles.

Les cellules humaines A549 sont mises en culture puis infectées à confluence par les virions précédents en respectant une multiplicité d'infection d'environ 100. Les quantités d'IL-2 secrétées dans les surnageants de culture récupérés 48 h après l'infection, sont déterminées par ELISA (trousse Quantikine hIL-2, R&D System, Minneapolis). Dans un contexte vecteur adénoviral de seconde génération (E1⁻, E3⁻ et E4⁻), l'IL-2 est produite en des quantités 100 à 150 fois plus élevées lorsque les virions portent une cassette d'expression munie d'un intron (AdTG6215) que lorsqu'ils en sont dépourvus (AdTG6692). Ces dernier synthétisent des niveaux d'IL-2 très faibles qui ne permettent pas d'envisager leur utilisation thérapeutique. En comparaison, le facteur d'amplification dans un contexte de virus de première génération (E1⁻, E3⁻) est de 2,5.

### EXEMPLE 2 : Construction du vecteur adénoviral AdTG9378 exprimant le gène codant pour le facteur IX canin.

Dans un premier temps, on reconstitue la cassette recombinante constituée du promoteur RSV, de la séquence d'épissage du second intron du gène β-globine de lapin placée en 5' de l'ADNc du FIX canin suivi du polyA du gène β-globine de lapin. Les séquences d'épissage et polyA β-globine sont excisées du vecteur pBCMGNeo (Karasuyama et al., 1989, *supra*) par digestion *Saf*I*-Bam*HI et clonées en aval du promoteur RSV porté par le fragment *Sal*I*-Bam*HI isolé du vecteur pREP4 (Invitrogen V004-50). Puis on insère en aval des séquences d'épissage, l'ADNc codant pour le FIX canin dont la séquence est décrite dans Evans et al. (1989, Blood 74, 207-212). L'unité de transcription est placée dans un vecteur de transfert qui contient les séquences Ad5 1 à 458 et 3328 à 5778, pour donner pTG9350. Le vecteur adénoviral pTG9378 est obtenu par recombinaison homologue entre le fragment *Pac*I*-Bgl*I isolé de pTG9350 et le vecteur pTG8595 (Chartier et al., 1996, *supra*) linéarisé par *CIa*I. Il contient le génome Ad5 dépourvu des régions E1 (nt 459 à 3327), E3 (nt 28592 à 30470) et E4 (nt 32994 à 34998) et la cassette FIX indiquée ci-dessus insérée en lieu et place de E1.

Comme précédemment , on construit un témoin désigné pTG5666 dont l'ossature adénovirale correspond à un vecteur de seconde génération mais dans lequel la cassette FIX est dépourvue de séquences d'épissage, par digestion *Pst*I.

De même, on construit deux vecteurs de première génération pTG9370 et pTG9383 différant respectivement par la présence ou non de l'intron 2β-globine dans la cassette FIX. Le premier est obtenu par recombinaison homologue entre le fragment *Pac*I*-Bgl*I isolé de pTG9350 et le vecteur pTG4656 délété des régions E1 (nt 459 à 3327) et E3 (28592 à 30470), linéarisé par *Cla*I*.* Le second résulte de la recombinaison entre le fragment *Pac*I*-Bgl*I dépourvu d'intron et pTG4656.

Les particules virales sont générées par transfection des fragments *Pac*I des plasmides précédents dans les lignées 293 (AdTG9370 et AdTG9383) ou LCA1 (AdTG9378 et AdTG5666). Les virus sont isolés, propagés et purifiés dans les conditions habituelles. Les cellules cibles A549 sont mises en culture puis, une fois à confluence, infectées par les virions précédents en respectant une multiplicité d'infection d'environ 100. Les quantités de FIX canins secrétées dans les surnageants de culture récupérés 48 h après l'infection sont déterminées par ELISA (voir par exemple Axelrod et al., 1990, Proc. Natl. Acad. Sci. USA *87*, 5173-5177; Roman et al., 1992, Somat. Cell Mol. Genet. *18*, 247-258 ; Miyanohara et al., 1992, New Biol. *4*, 238-246; Lozier et al., 1994, JAMA *271*, 47-51). Un test particulièrement approprié utilise à titre d'anticorps de capture, le monoclonal FXCOO8 (Bajaj et al., 1985, J. Biol. Chem. *260*, 11574-11580) à raison de 200 ng par puits et à titre d'anticorps de détection, un anticorps de lapin spécifique du FIX humain (STAGO) couplé à la péroxydase. La secrétion de FIX canin dans les cellules A549 infectées par les virus de seconde génération est 20 fois plus élevée lorsque la cassette comporte des séquences d'épissage (AdTG9378) que lorsqu'elle en est dépourvue (AdTG5666). Lorsque la transduction met en oeuvre des vecteurs de première génération, l'effet bénéfique de l'intron est moins marqué (facteur d'amplification d'environ 2,5).

Le remplacement de l'ADNc du FIX par celui codant pour l'IL-2humaine donne lieu au vecteur pTG6214. il est l'équivalent à l'AdTG9378 à la différence du gène d'intérêt.

### EXEMPLE 3 : Effet de l'intron sur la production virale.

Environ 10⁷ cellules A549-E1 #73 sont mises en culture dans des flasques F25 et sont infectées à une MOI de 1 avec les virus AdTG6624 (ΔE1ΔE3 +intron) ou AdTG6229 (ΔE1ΔE3 -intron). L'infection est réalisée 30 min à 37°C en milieu DMEM (Dulbecco's modified Eagle's médium) additionné de 2 % de sérum. Les cultures sont récoltées aux temps 24 h, 48 h, 72 h et 96 h et les virions libérés des cellules par chocs thermiques. Le titre viral est évalué par immunofluorescence de la protéine DBP à l'aide d'un anticorps monoclonal spécifique (Reich et al., 1983, Virology *128*, 480-484). Le facteur d'amplification est déterminé par le rapport du nombre d'unités infectieuses (u.i.) finales sur initiales. On observe un facteur d'amplification de l'ordre de 1900, 4100 et 3300 à 48, 72 et 96 h post infection respectivement avec les deux types de constructions. Lorsque la même expérience est conduite dans en flasques de 175 (MOI=3 et récolte à 3 jours post-infection), le rendement de production (i.u/cellule) est sensiblement plus élevé (d'environ 25 %) avec les virus AdTG6624 qui comportent une cassette d'expression munie d'un intron. Dans leur ensemble, ces résultats indiquent que la présence de l'intron synthétique de pCI n'a pas d'effet négatif sur les rendements de production virale.

### EXEMPLE 4 : Fonctionnalité des vecteurs in vitro et in vivo.

Divers types cellulaires aussi bien de lignées établies d'origines variées [humaine, simienne ou murine : A549, Vero et W162 (Weinberg et Ketner, 1983, Proc. Natl. Acad. Sci. USA 80, 5384-5386)] que de cellules primaires de souris (fibroblastes), canines (myoblastes) et surtout d'origine humaine [tumeurs primaires issues d'un cancer de l'estomac (passage 5) et d'une métastase hépatique d'un cancer de colon (passage 2)] sont transduites de façon standard à une moi de 50 à 100. Les virus uilisés sont les suivants : AdTG6624 (ΔE1ΔE3 pCMV+intron), AdTG6229 (ΔE1ΔE3 pCMV-intron), AdTG6215 (ΔE1ΔE3ΔE4 pCMV+intron) ou AdTG6692 (ΔE1ΔE3ΔE4 pCMV-intron), AdTG6214 (ΔE1ΔE3ΔE4 pRSV+intron) et son contrôle (ΔE1ΔE3ΔE4 pRSV-intron). Les surnageants sont récoltés 24 et 72 h après l'infection et les quantités d'IL-2 sécrétées sont déterminées par ELISA comme précédemment.

Les dosages indiquent l'effet bénéfique de l'intron en terme de production d'IL-2, celle ci étant 2 à 3 fois plus élevée dans un contexte vecteur de première génération et plus de 100 fois dans un contexte ΔE1ΔE3ΔE4. On note que les virions AdTG6624 sont les plus productifs en IL-2, montrant l'intérêt à associer le promoteur CMV, l'intron synthétique dans une ossature vecteur de première génération.

L'activité antitumorale des virions exprimant l'IL-2 est évaluée *in vivo* dans un modèle murin de tumeurs. Des souris femelles immunodéficientes B6D2 agées de 6 à 8 semaines sont rendues cancéreuses par administration de 3x10⁵ cellules tumorales P815. Une fois les tumeurs palpables (3 à 4 mm de diamètre), on inocule 5x10⁸ particules infectieuses d'AdTG6624 par voie intratumorale à J0, J1 et J2 et on suit la survie des animaux au cours du temps. Le pourcentage de survie des animaux traités avec les virus AdTG6624 atteint 40 % plus de 40 jours post-infection. En comparaison, les animaux traités avec un virus contrôle contenant une cassette d'expression de l'IL-2 sans intron dirigée par le promoteur MLP présentent un taux de survie beaucoup plus faible.

## Revendications

1. Vecteur adénoviral dérivant du génome d'un adénovirus au moins par délétion de tout ou partie de la région E1, ledit vecteur adénoviral comportant une cassette d'expression d'un gène d'intérêt placé sous le contrôle des éléments nécessaires à son expression dans une cellule hôte ou un organisme hôte, lesdits éléments nécessaires à l'expression comprenant au moins une séquence d'épissage, **caractérisé en ce que** ladite séquence d'épissage comprend un site donneur et un site accepteur d'épissage et dérive d'un gène β-globine, ou d'une séquence d'épissage synthétique ayant la séquence représentée à l'identificateur de séquence IDS1.

2. Vecteur adénoviral selon la revendication 1, **caractérisé en ce que** ladite séquence d'épissage dérive de l'intron 2 du gène β-globine de lapin ou d'une séquence d'épissage comprenant le site donneur d'épissage de l'intron 1 du gène β-globine humaine ainsi que le point de branchement et le site accepteur d'épissage du gène d'une immunoglobine de souris.

3. Vecteur adénoviral selon la revendication 2, **caractérisé en ce que** ladite séquence d'épissage comprend une séquence identique à au moins 70%, ou identique à la séquence représentée à l'identificateur de séquence IDS 1 ou 2.

4. Vecteur adénoviral selon la revendication 3, **caractérisé en ce que** ladite séquence d'épissage comprend une séquence sensiblement telle que représentée à l'identificateur de séquence IDS 1 ou 2.

5. Vecteur adénoviral selon l'une des revendications 1 à 4, **caractérisé en ce que** ladite séquence d'épissage est insérée dans ladite cassette d'expression entre le premier exon et le second exon du gène d'intérêt.

6. Vecteur adénoviral selon l'une des revendications 1 à 5, **caractérisé en ce que** ladite séquence d'épissage comprend à l'une et l'autre de ses extrémités une séquence exonique et est insérée dans ladite cassette d'expression en 5' ou 3' du gène d'intérêt, celui-ci étant de type ADNc.

7. Vecteur adénoviral selon l'une des revendications 1 à 6, **caractérisé en ce qu'**en outre l'une ou moins des régions sélectionnées parmi E2, E4, L1, L2, L3, L4 et L5 est non fonctionnelle.

8. Vecteur adénoviral selon l'une des revendications 1 à 7, **caractérisé en ce qu'**il est en outre dépourvu de tout ou partie de la région E3.

9. Vecteur adénoviral selon l'une des revendications 1 à 8, sélectionné parmi le groupe suivant :
(1) vecteur adénoviral dépourvu de tout ou partie des régions E 1 et E2 et, de manière optionnelle, de tout ou partie de la région E3,
(2) vecteur adénoviral dépourvu de tout ou partie des régions E1 et E4 et, de manière optionnelle, de tout ou partie de la région E3,
(3) vecteur adénoviral dépourvu de tout ou partie des régions E1 et E4 et, de manière optionnelle, de tout ou partie de la région E3, et comportant une mutation non fonctionnelle dans la région E2,
(4) vecteur adénoviral dépourvu de tout ou partie des régions E1, E2 . et E4 et, de manière optionnelle de tout ou partie de la région E3,
(5) vecteur adénoviral dépourvu de tout ou partie de la région E1 et, de manière optionnelle de tout ou partie de la région E3.

10. Vecteur adénoviral selon l'une des revendications 1 à 9, **caractérisé en ce qu'**il dérive du génome d'un adénovirus d'origine humaine, canine, aviaire, bovine, murine, ovine, porcine ou simienne ou encore d'un hybride comprenant des fragments de génome adénoviral de différentes origines.

11. Vecteur adénoviral selon la revendication 10, **caractérisé en ce qu'**il dérive du génome d'un adénovirus humain de type 5.

12. Vecteur adénoviral selon l'une des revendications 1 à 11, **caractérisé en ce que** le gène d'intérêt code pour un ARN antisens, un ribosyme ou un polypeptide d'intérêt thérapeutique sélectionné parmi une cytokine, un récepteur cellulaire, un ligand, un facteur de coagulation, la protéine CFTR, l'insuline, la dystrophine, un facteur de croissance, une enzyme, un inhibiteur d'enzyme, un polypeptide à effet anti-tumoral, un inhibiteur d'angiogénèse, un polypeptide capable d'inhiber une infection bactérienne, parasitaire ou vitale et, notamment le HIV, un anticorps, une toxine, une immunotoxine et un marqueur.

13. Vecteur adénoviral selon l'une des revendications 1 à 12, **caractérisé en ce que** lesdits éléments nécessaires à l'expression dudit gène d'intérêt dans une cellule ou un organisme hôte comprennent un promoteur, notamment sélectionné parmi les promoteurs MLP (Major Late Promoter), PGK (Phospho Glycérate Kinase), RSV (Rous sarcoma Virus), SRα et CMV (Cytomégalovirus).

14. Vecteur adénoviral selon l'une des revendications 1 à 13, **caractérisé en ce que** lesdits éléments nécessaires à l'expression dudit gène d'intérêt dans une cellule ou un organisme hôte comprennent une séquence de polyadénylation, notamment dérivée du virus SV40 ou du gène β-globine de lapin.

15. Particule virale infectieuse comprenant un vecteur adénoviral selon l'une des revendications 1 à 14.

16. Cellule hôte eucaryote comprenant un vecteur adénoviral selon l'une des revendications 1 à 14 ou infectée par une particule virale infectieuse selon la revendication 15.

17. Procédé de préparation d'une particule virale infectieuse selon la revendication 15, selon lequel :
(i) on introduit un vecteur adénoviral selon l'une des revendications 1 à 14 dans une cellule de complémentation capable de complémenter *en trans* ledit vecteur adénoviral pour obtenir une cellule de complémentation transfectée;
(ii) on cultive ladite cellule de complémentation transfectée dans des conditions appropriées pour permettre la production de ladite particule virale infectieuse; et
(iii) on récupère ladite particule virale infectieuse dans la culture cellulaire.

18. Composition pharmaceutique comprenant à titre d'agent thérapeutique ou prophylactique un vecteur adénoviral selon l'une des revendications 1 à 14, une particule virale infectieuse selon la revendication 15 ou susceptible d'être obtenue en mettant en oeuvre un procédé de préparation selon la revendication 17 ou une cellule hôte eucaryote selon la revendication 16, en association avec un support acceptable d'un point de vue pharmaceutique.

19. Utilisation d'un vecteur adénoviral selon l'une des revendications 1 à 14, d'une particule virale infectieuse selon la revendication 15 ou susceptible d'être obtenue en mettant en oeuvre un procédé de préparation selon la revendication 17 ou d'une cellule hôte eucaryote selon la revendication 16 et dans lequel ou laquelle ledit gène d'intérêt est une copie fonctionnelle du gène codant pour la dystrophine, pour la préparation d'un médicament destiné au traitement préventif ou curatif des myopathies de Duchenne et Becker.

20. Utilisation d'un vecteur adénoviral selon l'une des revendications 1 à 14, d'une particule virale infectieuse selon la revendication 15 ou susceptible d'être obtenue en mettant en oeuvre un procédé de préparation selon la revendication 17 ou d'une cellule hôte eucaryote selon la revendication 16, et dans lequel ou laquelle ledit gène d'intérêt code pour un produit cytotoxique, pour la préparation d'un médicament destiné au traitement de cancers, ledit produit cytotoxique étant choisi parmi, la thymidine kinase du virus de simplex de l'herpès (TK-HSV-1), la ricine, la toxine cholérique ou diphtérique, le produit d'expression des gènes de levure *FCY1* et *FUR1* codant pour l'uracile phosphoribosyl transférase et la cytosine désaminase, le produit d'expression du gène suppresseur de tumeur p53, Rb ou p73.

21. Utilisation d'un vecteur adénoviral selon l'une des revendications 1 à 14, d'une particule virale infectieuse selon la revendication 15 ou susceptible d'être obtenue en mettant en oeuvre un procédé de préparation selon la revendication 17 ou d'une cellule hôte eucaryote selon la revendication 16, dans lequel ou laquelle ledit gène d'intérêt code pour un antigène associé à une tumeur, éventuellement en combinaison avec un gène codant pour une cytokine, pour la préparation d'un médicament destiné au traitement de cancers, le dit antigène étant choisi parmi MUC-1, BRCA-1, les antigènes précoces ou tardifs E6, E7, L1 et L2 d'un virus à papillome HPV.

22. Utilisation d'un vecteur adénoviral selon l'une des revendications 1 à 14, d'une particule virale infectieuse selon la revendication 15 ou susceptible d'être obtenue en mettant en oeuvre un procédé de préparation selon la revendication 17 ou d'une cellule hôte eucaryote selon la revendication 16, dans lequel ou laquelle ledit gène d'intérêt code pour l'IL-2, pour la préparation d'un médicament destiné au traitement de cancers.

## Claims

1. Adenoviral vector which is derived from the genome of an adenovirus at least by deleting all or part of the E1 region, with the said adenoviral vector containing a cassette for expressing a gene of interest which is placed under the control of the elements which are required for expressing it in a host cell or organism, with the said elements which are required for the expression comprising at least one splicing sequence, **characterized in that** the said splicing sequence comprises a donor site and a splicing acceptor site and is derived from a β-globin gene, or from a synthetic splicing sequence having the sequence depicted in the IDS 1 sequence identifier.

2. Adenoviral vector according to Claim 1, **characterized in that** the said splicing sequence is derived from intron 2 of the rabbit β-globin gene or from a splicing sequence which comprises the splicing donor site of intron 1 of the human β-globin gene as well as the branching point and the splicing acceptor site of the gene for a mouse immunoglobulin.

3. Adenoviral vector according to Claim 2, **characterized in that** the said splicing sequence comprises a sequence which is at least 70% identical to, or identical to, the sequence depicted in the IDS 1 or 2 sequence identifier.

4. Adenoviral vector according to Claim 3, **characterized in that** the said splicing sequence comprises a sequence which is substantially as depicted in the IDS 1 or 2 sequence identifier.

5. Adenoviral vector according to one of Claims 1 to 4, **characterized in that** the said splicing sequence is inserted into the said expression cassette between the first exon and the second exon of the gene of interest.

6. Adenoviral vector according to one of Claims 1 to 5, **characterized in that** the said splicing sequence comprises an exon sequence at one or other of its ends and is inserted into the said expression cassette 5' or 3' of the gene of interest, with the gene being of the cDNA type.

7. Adenoviral vector according to one of Claims 1 to 6, **characterized in that** at least one of the regions selected from E2, E4, L1, L2, L3, L4 and L5 is also nonfunctional.

8. Adenoviral vector according to one of Claims 1 to 7, **characterized in that** it additionally lacks all or part of the E3 region.

9. Adenoviral vector according to one of Claims 1 to 8, which is selected from the following group:
(1) adenoviral vector which lacks all or part of the E1 and E2 regions and, optionally, all or part of the E3 region,
(2) adenoviral vector which lacks all or part of the E1 and E4 regions and, optionally, all or part of the E3 region,
(3) adenoviral vector which lacks all or part of the E1 and E4 regions and, optionally, all or part of the E3 region, and which contains a nonfunctional mutation in the E2 region,
(4) adenoviral vector which lacks all or part of the E1, E2 and E4 regions and, optionally, all or part of the E3 region,
(5) adenoviral vector which lacks all or part of the E1 region and, optionally, all or part of the E3 region.

10. Adenoviral vector according to one of Claims 1 to 9, **characterized in that** it is derived from the genome of an adenovirus of human, canine, avian, bovine, murine, ovine, porcine or simian origin or else from a hybrid which comprises adenoviral genome fragments of different origins.

11. Adenoviral vector according to Claim 10, **characterized in that** it is derived from the genome of a human type 5 adenovirus.

12. Adenoviral vector according to one of Claims 1 to 11, **characterized in that** the gene of interest encodes an antisense RNA, a ribosyme or a therapeutic polypeptide of therapeutic interest which is selected from a cytokine, a cell receptor, a ligand, a coagulation factor, the CFTR protein, insulin, dystrophin, a growth factor, an enzyme, an enzyme inhibitor, a polypeptide having an anti-neoplastic effect, an angiogenesis inhibitor, a polypeptide which is able to inhibit a bacterial, parasitic or viral, in particular HIV, infection, an antibody, a toxin, an immunotoxin and a label.

13. Adenoviral vector according to one of Claims 1 to 12, **characterized in that** the said elements which are required for expressing the said gene of interest in a host cell or organism comprise a promoter which is selected, in particular, from the MLP (Major Late Promoter), PGK (Phospho Glycerate Kinase), RSV (Rous Sarcoma Virus), SRα and CMV (Cytomegalovirus) promoters.

14. Adenoviral vector according to one of Claims 1 to 13, **characterized in that** the said elements which are required for expressing the said gene of. interest in a host cell or organism comprise a polyadenylation sequence, in particular derived from the SV40 virus or the rabbit β-globin gene.

15. Infectious viral particle which comprises an adenoviral vector according to one of Claims 1 to 14.

16. Eukaryotic host cell which comprises an adenoviral vector according to one of Claims 1 to 14 or which is infected with an infectious viral particle according to Claim 15.

17. Process for preparing an infectious viral particle according to Claim 15, according to which:
(i) an adenoviral vector according to one of Claims 1 to 14 is introduced into a complementing cell which is able to complement the said adenoviral vector *in trans* so as to obtain a transfected complementing cell;
(ii) the said transfected complementing cell is cultured under conditions which are appropriate for enabling the said infectious viral particle to be produced; and
(iii) the said infectious viral particle is recovered from the cell culture.

18. Pharmaceutical composition which comprises, as a therapeutic or prophylactic agent, an adenoviral vector according to one of Claims 1 to 14, an infectious viral particle which is in accordance with Claim 15 or which can be obtained by implementing a preparation process according to Claim 17, or a eukaryotic host cell according to Claim 16, in combination with an excipient which is acceptable from the pharmaceutical point of view.

19. Use of an adenoviral vector according to one of Claims 1 to 14, of an infectious viral particle which is in accordance with Claim 15 or which can be obtained by implementing a preparation process according to Claim 17, or of a eukaryotic host cell according to Claim 16, and in which the said gene of interest is a functional copy of the gene encoding dystrophin, for preparing a medicament which is intended for the preventive or curative treatment of Duchenne and Becker myopathies.

20. Use of an adenoviral vector according to one of Claims 1 to 14, of an infectious viral particle which is in accordance with Claim 15 or which can be obtained by implementing a preparation process according to Claim 17, or of a eukaryotic host cell according to Claim 16, and in which the said gene of interest encodes a cytotoxic product, for preparing a medicament which is intended for treating cancers, the said cytotoxic product being selected from: herpes simplex virus thymidine kinase (HSV-1 TK), ricin, cholera toxin or diphtheria toxin, the expression product of the yeast genes *FCY1* and *FUR1,* encoding uracil phosphoribosyl transferase and cytosine deaminase, the expression product of the tumour suppressor gene p53, Rb or p73.

21. Use of an adenoviral vector according to one of Claims 1 to 14, of an infectious viral particle which is in accordance with Claim 15 or which can be obtained by implementing a preparation process according to Claim 17, or of a eukaryotic host cell according to Claim 16, in which the said gene of interest encodes a tumour-associated antigen, where appropriate in combination with a gene encoding a cytokine, for preparing a medicament which is intended for treating cancers, the said antigen being selected from MUC-1, BRCA-1 and the early or late antigens E6, E7, L1 and L2 of an HPV papilloma virus.

22. Use of an adenoviral vector according to one of Claims 1 to 14, of an infectious viral particle which is in accordance with Claim 15 or which can be obtained by implementing a preparation process according to Claim 17, or of a eukaryotic host cell according to Claim 16, in which the said gene of interest encodes IL-2, for preparing a medicament which is intended for treating cancers.

## Patentansprüche

1. Adenoviraler Vektor, welcher sich von dem Genom eines Adenovirus wenigstens durch Deletion der Gesamtheit oder eines Teils der E1-Region ableitet, wobei der adenovirale Vektor eine Expressionskassette eines Gens von Interesse, welches unter die Kontrolle der für dessen Expression in einer Wirtszelle oder einem Wirtsorganismus erforderlichen Elemente gestellt ist, umfasst, wobei die für die Expression erforderlichen Elemente wenigstens eine Spleißsequenz umfassen, **dadurch gekennzeichnet, dass** die Spleißsequenz eine Spleißdonorstelle und eine Spleißakzeptorstelle umfasst und sich von einem β-Globin-Gen oder einer synthetischen Spleißsequenz, welche die in der Sequenzbeschreibung IDS 1 angegebene Sequenz aufweist, ableitet.

2. Adenoviraler Vektor nach Anspruch 1, **dadurch gekennzeichnet, dass** die Spleißsequenz sich von dem Intron 2 des β-Globin-Gens des Kaninchens oder von einer Spleißsequenz, welche die Spleißdonorstelle des Introns 1 des humanen β-Globin-Gens sowie den Verzweigungspunkt und die Spleißakzeptorstelle des Gens eines Immunglobulins von der Maus umfasst, ableitet.

3. Adenoviraler Vektor nach Anspruch 2, **dadurch gekennzeichnet, dass** die Spleißsequenz eine Sequenz umfasst, welche zu wenigstens 70% identisch ist oder identisch ist mit der in der Sequenzbeschreibung IDS 1 oder 2 angegebenen Sequenz.

4. Adenoviraler Vektor nach Anspruch 3, **dadurch gekennzeichnet, dass** die Spleißsequenz eine Sequenz umfasst, welche im wesentlichen so ist, wie in der Sequenzbeschreibung IDS 1 oder 2 angegeben.

5. Adenoviraler Vektor nach einem der Ansprüche 1 bis 4, **dadurch gekennzeichnet, dass** die Spleißsequenz in die Expressionskassette zwischen dem ersten Exon und dem zweiten Exon des Gens von Interesse inseriert ist.

6. Adenoviraler Vektor nach einem der Ansprüche 1 bis 5, **dadurch gekennzeichnet, dass** die Spleißsequenz an ihren beiden Enden eine Exonsequenz umfasst und in die Expressionskassette 5' oder 3' von dem Gen von Interesse, welches vom Typ cDNA ist, inseriert ist.

7. Adenoviraler Vektor nach einem der Ansprüche 1 bis 6, **dadurch gekennzeichnet, dass** außerdem wenigstens eine der Regionen, ausgewählt unter E2, E4, L1, L2, L3, L4 und L5, nicht funktional (funktionsfähig) ist.

8. Adenoviraler Vektor nach einem der Ansprüche 1 bis 7, **dadurch gekennzeichnet, dass** diesem außerdem die Gesamtheit oder ein Teil der Region E3 fehlt.

9. Adenoviraler Vektor nach einem der Ansprüche 1 bis 8, welcher aus der folgenden Gruppe ausgewählt wird:
(1) adenoviraler Vektor, welchem die Gesamtheit oder ein Teil der Regionen E1 und E2 und gegebenenfalls die Gesamtheit oder ein Teil der Region E3 fehlt,
(2) adenoviraler Vektor, welchem die Gesamtheit oder ein Teil der Regionen E1 und E4 und gegebenenfalls die Gesamtheit oder ein Teil der Region E3 fehlt,
(3) adenoviraler Vektor, welchem die Gesamtheit oder ein Teil der Regionen E1 und E4 und gegebenenfalls die Gesamtheit oder ein Teil der Region E3 fehlt und welcher eine nichtfunktionale Mutation in der Region E2 umfasst,
(4) adenoviraler Vektor, welchem die Gesamtheit oder ein Teil der Regionen E1, E2 und E4 und gegebenenfalls die Gesamtheit oder ein Teil der Region E3 fehlt,
(5) adenoviraler Vektor, welchem die Gesamtheit oder ein Teil der Region E1 und gegebenenfalls die Gesamtheit oder ein Teil der Region E3 fehlt.

10. Adenoviraler Vektor nach einem der Ansprüche 1 bis 9, **dadurch gekennzeichnet, dass** er sich von dem Genom eines Adenovirus von humanem Ursprung, Hunde-, Vogel-, Rinder-, Maus-, Schaf-, Schweine- oder Affenursprung oder ferner von einem Hybrid, welches Fragmente von adenoviralen Genomen unterschiedlicher Ursprünge umfasst, ableitet.

11. Adenoviraler Vektor nach Anspruch 10, **dadurch gekennzeichnet, dass** er sich von dem Genom eines humanen Adenovirus vom Typ 5 ableitet.

12. Adenoviraler Vektor nach einem der Ansprüche 1 bis 11, **dadurch gekennzeichnet, dass** das Gen von Interesse eine Antisinn-RNA, ein Ribozym oder ein Polypeptid von therapeutischem Interesse, ausgewählt aus einem Zytokin, einem zellulären Rezeptor, einem Ligand, einem Gerinnungsfaktor, dem CFTR-Protein, Insulin, Dystrophin, einem Wachstumsfaktor, einem Enzym, einem Enzyminhibitor, einem Polypeptid mit antitumoraler Wirkung, einem Angiogenese-Inhibitor, einem Polypeptid, welches in der Lage ist, eine bakterielle, durch Parasiten verursachte oder virale Infektion und insbesondere HIV zu hemmen, einem Antikörper, einem Toxin, einem Immuntoxin und einem Marker, kodiert.

13. Adenoviraler Vektor nach einem der Ansprüche 1 bis 12, **dadurch gekennzeichnet, dass** die für die Expression des Gens von Interesse in einer Wirtszelle oder einem Wirtsorganismus erforderlichen Elemente einen Promotor, welcher insbesondere unter den MLP- (Major Late Promoter), PGK- (Phosphoglyceratkinase), RSV- (Rous-Sarkom-Virus), SRα- und CMV- (Zytomegalievirus) -Promotoren ausgewählt wird, umfassen.

14. Adenoviraler Vektor nach einem der Ansprüche 1 bis 13, **dadurch gekennzeichnet, dass** die für die Expression des Gens von Interesse in einer Wirtszelle oder einem Wirtsorganismus erforderlichen Elemente eine Polyadenylierungssequenz, welche insbesondere von dem SV40-Virus oder dem β-Globin-Gen des Kaninchens abgeleitet ist, umfassen.

15. Infektiöses Viruspartikel, welches einen adenoviralen Vektor nach einem der Ansprüche 1 bis 14 umfasst.

16. Eukaryotische Wirtszelle, welche einen adenoviralen Vektor nach einem der Ansprüche 1 bis 14 umfasst oder durch ein infektiöses Viruspartikel nach Anspruch 15 infiziert ist.

17. Verfahren zur Herstellung eines infektiösen Viruspartikels nach Anspruch 15, gemäß welchem:
(i) man einen adenoviralen Vektor nach einem der Ansprüche 1 bis 14 in eine Komplementationszelle, welche in der Lage ist, den adenoviralen Vektor in trans zu komplementieren, einschleust, um eine transfizierte Komplementationszelle zu erhalten;
(ii) man die transfizierte Komplementationszelle unter geeigneten Bedingungen kultiviert, um die Produktion des infektiösen Viruspartikels zu ermöglichen; und
(iii) man das infektiöse Viruspartikel in der Zellkultur gewinnt.

18. Pharmazeutische Zusammensetzung, welche als therapeutisches oder prophylaktisches Agens einen adenoviralen Vektor nach einem der Ansprüche 1 bis 14, ein infektiöses Viruspartikel nach Anspruch 15 oder welches erhalten werden kann, indem man ein Herstellungsverfahren nach Anspruch 17 ausführt, oder eine eukaryotische Wirtszelle nach Anspruch 16 in Kombination mit einem aus pharmazeutischer Sicht annehmbaren Träger umfasst.

19. Verwendung eines adenoviralen Vektors nach einem der Ansprüche 1 bis 14, eines infektösen Viruspartikels nach Anspruch 15 oder welches erhalten werden kann, indem man ein Herstellungsverfahren nach Anspruch 17 ausführt, oder einer eukaryotischen Wirtszelle nach Anspruch 16 und bei welchem oder bei welcher das Gen von Interesse eine funktionale Kopie des Dystrophin kodierenden Gens ist, für die Herstellung eines Arzneimittels, welches für die präventive oder heilende Behandlung von Duchenneund Becker-Muskeldystrophie bestimmt ist.

20. Verwendung eines adenoviralen Vektors nach einem der Ansprüche 1 bis 14, eines infektösen Viruspartikels nach Anspruch 15 oder welches erhalten werden kann, indem man ein Herstellungsverfahren nach Anspruch 17 ausführt, oder einer eukaryotischen Wirtszelle nach Anspruch 16 und bei welchem oder bei welcher das Gen von Interesse ein zytotoxisches Produkt kodiert, für die Herstellung eines Arzneimittels, welches für die Behandlung von Krebs bestimmt ist, wobei das zytotoxische Produkt ausgewählt wird unter: der Thymidinkinase des Herpes simplex-Virus (TK-HSV-1), Ricin, Cholera- oder Diphtherietoxin, dem Expressionsprodukt der Hefe-Gene *FCY1* und *FUR1,* welche Uracilphosphoribosyltransferase und Cytosindesaminase kodieren, dem Expressionsprodukt des Tumorsuppressorgens p53, Rb oder p73.

21. Verwendung eines adenoviralen Vektors nach einem der Ansprüche 1 bis 14, eines infektösen Viruspartikels nach Anspruch 15 oder welches erhalten werden kann, indem man ein Herstellungsverfahren nach Anspruch 17 ausführt, oder einer eukaryotischen Wirtszelle nach Anspruch 16, bei welchem oder bei welcher das Gen von Interesse ein mit einem Tumor assoziiertes Antigen kodiert, gegebenenfalls in Kombination mit einem Gen, welches ein Zytokin kodiert, für die Herstellung eines Arzneimittels, welches für die Behandlung von Krebs bestimmt ist, wobei das Antigen unter MUC-1, BRCA-1, den frühen oder späten Antigenen E6, E7, L1 und L2 eines HPV-Papillomavirus ausgewählt wird.

22. Verwendung eines adenoviralen Vektors nach einem der Ansprüche 1 bis 14, eines infektösen Viruspartikels nach Anspruch 15 oder welches erhalten werden kann, indem man ein Herstellungsverfahren nach Anspruch 17 ausführt, oder einer eukaryotischen Wirtszelle nach Anspruch 16, bei welchem oder bei welcher das Gen von Interesse IL-2 kodiert, für die Herstellung eines Arzneimittels, welches für die Behandlung von Krebs bestimmt ist.
